(19) Europäisches Patentamt
European Patent Office
Office européen des brevets

(11) **EP 4 692 339 A1**

(12) **EUROPEAN PATENT APPLICATION**
published in accordance with Art. 153(4) EPC

(43) Date of publication:
**11.02.2026 Bulletin 2026/07**

(21) Application number: **24803757.4**

(22) Date of filing: **09.05.2024**

(51) International Patent Classification (IPC):
***C12N 9/24*** *(2006.01)* ***C12N 1/20*** *(2026.01)*
***C12N 1/38*** *(2006.01)*

(52) Cooperative Patent Classification (CPC):
**C12N 1/20; C12N 1/38; C12N 9/24**

(86) International application number:
**PCT/KR2024/006294**

(87) International publication number:
**WO 2024/232704 (14.11.2024 Gazette 2024/46)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **10.05.2023 KR 20230060588**

(71) Applicant: **CJ Cheiljedang Corporation**
**Seoul 04560 (KR)**

(72) Inventors:
- **LEE, Hyo Hyoung**
  **Seoul 04560 (KR)**
- **LEE, Sungkyun**
  **Seoul 04560 (KR)**
- **KIM, Taekbeom**
  **Seoul 04560 (KR)**
- **PARK, Jung Won**
  **Seoul 04560 (KR)**
- **KIM, Young Kyung**
  **Seoul 04560 (KR)**
- **KIM, Yu Shin**
  **Seoul 04560 (KR)**
- **KIM, Minhoe**
  **Seoul 04560 (KR)**

(74) Representative: **Meissner Bolte Partnerschaft mbB**
**Patentanwälte Rechtsanwälte**
**Postfach 86 06 24**
**81633 München (DE)**

Remarks:
The complete document including Reference Table(s) and the Sequence Listing(s) can be downloaded from the EPO website

(54) **MEDIUM COMPOSITION FOR IMPROVING PECTINASE PRODUCTIVITY OF BACILLUS SPP. AND METHOD FOR PRODUCING PECTINASE BY USING SAME**

(57) The present invention relates to a medium composition for enhancing the pectinase productivity of a Bacillus sp. strain and a method for producing a pectinase by using same.

【FIG. 3】

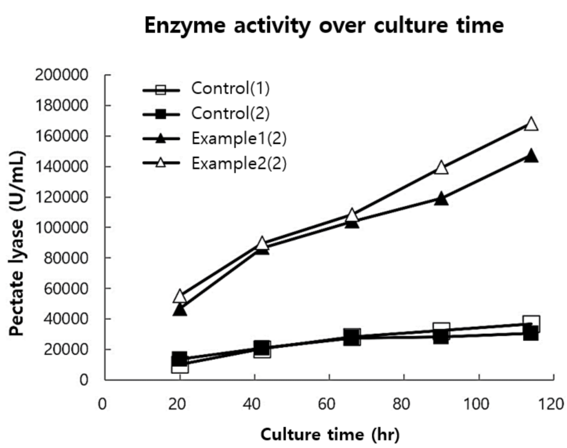


EP 4 692 339 A1

## Description

[TECHNICAL FIELD]

Cross-Reference to Related Application(s)

This application claims a benefit of priority based on Korean Patent Application No. 10-2023-0060588, filed on May 10, 2023, the entire contents of which are incorporated herein by reference.

The present disclosure relates to a medium composition for improving pectinase productivity of *Bacillus spp.* strains and a method for producing pectinase using the same.

[BACKGROUND ART]

Pectin is a substance widely distributed in the plant kingdom, forming a major component of the thin membrane that helps adjacent cells adhere to each other without separating. Enzymatic degradation of pectin components is a very important step in many industries such as food, textile, paper processing and the like. Enzymes that degrade pectin are generally referred to as pectinases and are classified according to their action mechanism into pectate lyase (EC 4.2.2.2), polygalacturonase (EC 3.2.1.15), exo-polygalacturonase (EC 3.2.1.67), pectin lyase (EC 4.2.2.10), and the like.

Pectate lyase, a type of pectinase, is an enzyme catalyzing the cleavage of $\alpha$-1,4 linkages from the side of the free carboxyl group of polygalacturonic acid which is produced from de-esterification of pectinaceous substances. It has been found to play a critical role in the development of plant soft root rot in conjunction with other pectinases, and thus closely related to the plant defense mechanism. Furthermore, recently, it has been utilized as a primary treatment agent as an important enzyme for removing pectinaceous substances in plant-derived water pollutants, and is also employed in biochemical pulp manufacturing and a food/feed additive.

Pectinases, including pectate lyase, are known to be produced by various microorganisms such as bacteria, yeasts, fungi, and actinomycetes, and can be mass-produced through microbial culture. Industrial production of pectinase is primarily carried out by *Aspergillus spp.* strains and *Bacillus spp.* strains.

Korean Laid-Open Patent Publication No. 10-2006-0074794 discloses a method for producing high-activity pectinase from *Aspergillus spp.* strains; but it does not disclose a method for producing high-activity pectinase from *Bacillus spp.* strains by optimizing the organic nitrogen sources in fermentation medium and feed medium for fed-batch culture.

[DISCLOSURE]

[TECHNICAL PROBLEM]

An object of the present disclosure is to provide a method for producing pectinase from a *Bacillus spp.* microorganism, the method comprising: (1) culturing a *Bacillus spp.* microorganism in a fermentation medium including soy peptone and yeast extract as nitrogen sources; and (2) supplying the medium of step (1) with a feed medium including soy peptone and yeast extract as nitrogen sources.

Another object of the present disclosure is to provide a composition for producing pectinase, comprising a fermentation medium and/or a feed medium including both soy peptone and yeast extract as nitrogen sources.

[TECHNICAL SOLUTION]

Hereinafter, the present invention will be described in detail. Meanwhile, each description and embodiment disclosed in the present disclosure can be applied to other descriptions and embodiments. That is, all combinations of elements disclosed in the present disclosure fall within the scope of the present disclosure. Furthermore, the scope of the present disclosure should not be regarded as limited by the specific descriptions provided below. In addition, those skilled in the art can recognize or ascertain numerous equivalents for specific aspects of the present disclosure described herein using only routine experimentation. Furthermore, such equivalents are intended to be included within the scope of the present disclosure.

An aspect of the present disclosure provides a method for producing pectinase from a *Bacillus spp.* microorganism, comprising: (1) a step of culturing a *Bacillus spp.* microorganism in a fermentation medium comprising soy peptone and yeast extract as nitrogen sources; and

(2) a step of supplying the medium of step (1) with a feed medium including soy peptone and yeast extract as nitrogen sources.

The method for producing pectinase from a *Bacillus spp.* microorganism of the present disclosure includes step (1): culturing a *Bacillus spp.* microorganism in a fermentation medium comprising soy peptone and yeast extract as nitrogen sources.

In the present disclosure, the *Bacillus spp.* microorganism may be *Bacillus subtilis, Bacillus licheniformis*, *Bacillus amyloliquefaciens*, or *Bacillus velezensis*, and more specifically, may be *Bacillus subtilis,* but is not limited thereto.

In the present disclosure, the pectinase may be pectate lyase, polygalacturonase, exo-polygalacturonase, or pectin lyase.

In the present disclosure, the term "pectate lyase" refers to an enzyme that catalyzes the cleavage of $\alpha$-1,4 linkages from the side of the free carboxyl group of polygalacturonic acid which is produced from de-esterification of pectinaceous substances, and has EC number of 4.2.2.2. The systematic name for "pectate lyase" is "(1$\rightarrow$4)-alpha-D-galacturonan lyase", and it may also be referred to as "pectate trans-eliminase", polygalacturonate lyase, pectic acid lyase, and may be abbreviated as "Pel".

In the present disclosure, the culture may be fed-batch culture.

In the present disclosure, the term "fed-batch culture" refers to a culturing method in which a feed medium is supplied after culturing has begun with a fermentation medium or a basal medium. Fed-batch culture has the advantage that a concentration of substrate in the culture broth can be arbitrarily controlled, and that the substrate is added at an appropriate rate, and does not flow out, allowing for free control over an amount of supplied substrate.

In the present disclosure, the term "medium" refers to a substance mixed with nutrients required for culturing microorganisms as main ingredients, supplying water indispensable for survival and growth, as well as nutrients and growth factors. Specifically, the medium and other culture conditions used for microbial culture in the present disclosure can be used without particular limitation, as long as they are commonly used for microbial culture.

In the present disclosure, the term "fermentation medium" refers to a medium that receives a feed medium (solution of supplying with substrate) in a fed-batch culture method, and may be used interchangeably with the terms "culture medium", "basal medium", "initial medium", or "main medium" herein.

The fermentation medium of step (1) may be a medium comprising soy peptone and yeast extract as nitrogen sources to increase the produced amount of pectinase from the *Bacillus spp.* microorganism or to increase the activity of pectinase produced from the *Bacillus spp.* microorganism.

Soy peptone included in the fermentation medium of the present disclosure is a protein derived from soybeans, primarily used as a nutrient source in microbial media, and contains about 90% of the protein content of isolated soy protein and contains the most globulin. It is one of the representative soybean protein food products along with concentrated soy protein and textured soy protein.

The content of soy peptone included in the fermentation medium of step (1) may be greater than 5 g/L, 6 g/L or more, 10 g/L or more, 15 g/L or more, 20 g/L or more, 6 to 49 g/L, 8 to 49 g/L, 10 to 49 g/L, 12 to 49 g/L, 14 to 49 g/L, 16 to 49 g/L, 18 to 49 g/L, 19 to 49 g/L, 6 to 45 g/L, 8 to 45 g/L, 10 to 45 g/L, 12 to 45 g/L, 14 to 45 g/L, 16 to 45 g/L, 18 to 45 g/L, 19 to 45 g/L, 6 to 40 g/L, 8 to 40 g/L, 10 to 40 g/L, 12 to 40 g/L, 14 to 40 g/L, 16 to 40 g/L, 18 to 40 g/L, 19 to 40 g/L, 6 to 35 g/L, 8 to 35 g/L, 10 to 35 g/L, 12 to 35 g/L, 14 to 35 g/L, 16 to 35 g/L, 18 to 35 g/L, 19 to 35 g/L, 6 to 30 g/L, 8 to 30 g/L, 10 to 30 g/L, 12 to 30 g/L, 14 to 30 g/L, 16 to 30 g/L, 18 to 30 g/L, 19 to 30 g/L, 6 to 28 g/L, 8 to 28 g/L, 10 to 28 g/L, 12 to 28 g/L, 14 to 28 g/L, 16 to 28 g/L, 18 to 28 g/L, 19 to 28 g/L, 6 to 26 g/L, 8 to 26 g/L, 10 to 26 g/L, 12 to 26 g/L, 14 to 26 g/L, 16 to 26 g/L, 18 to 26 g/L, 19 to 26 g/L, 10 to 24 g/L, 12 to 24 g/L, 14 to 24 g/L, 16 to 24 g/L, 18 to 24 g/L, 19 to 24 g/L, 10 to 22 g/L, 12 to 22 g/L, 14 to 22 g/L, 16 to 22 g/L, 18 to 22 g/L, 19 to 22 g/L, 10 to 21 g/L, 12 to 21 g/L, 14 to 21 g/L, 16 to 21 g/L, 18 to 21 g/L, or 19 to 21 g/L.

The content of yeast extract included in the fermentation medium of step (1) may be 5 to 15 g/L, 7.5 to 15 g/L, 9 to 15 g/L, 9.5 to 15 g/L, 5 to 12.5 g/L, 7.5 to 12.5 g/L, 9 to 12.5 g/L, 9.5 to 12.5 g/L, 5 to 11 g/L, 7.5 to 11 g/L, 9 to 11 g/L, 9.5 to 11 g/L, 5 to 10.5 g/L, 7.5 to 10.5 g/L, 9 to 10.5 g/L, or 9.5 to 10.5 g/L.

In an embodiment of the present disclosure, when being cultured in a fermentation medium containing soy peptone at a concentration greater than 5 g/L to less than 50 g/L, specifically 6 to 49 g/L based on a concentration of 10 g/L of yeast extract included a fermentation medium, it was confirmed that the activity of pectinase produced from the *Bacillus spp.* microorganism increased by 5% or more, 8% or more, 15% or more, 20% or more, 25% or more, 30% or more, 31% or more, 32% or more, 33% or more, or 34% or more.

Accordingly, the fermentation medium of step (1) may comprise soy peptone and yeast extract in a mixing ratio of 3:1 to 1:1, 2.5:1 to 1.5:1, and specifically 2:1.

The fermentation medium of step (1) may comprise a carbohydrate as a carbon source, and the carbon source may be at least one selected from the group consisting of glucose, fructose, maltose, galactose, mannose, sucrose, arabinose, xylose, and glycerol, and in an embodiment, the carbon source may be maltose and glucose.

The carbon source included in the fermentation medium of step (1) may be 10 to 50 g/L, 10 to 40 g/L, 10 to 35 g/L, 15 to 50 g/L, 15 to 40 g/L, 15 to 35 g/L, 20 to 50 g/L, 20 to 40 g/L, 20 to 35 g/L, 25 to 50 g/L, 25 to 40 g/L, or 25 to 35 g/L, and when the carbon source is maltose and glucose, the mixing ratio of maltose to glucose may be 1:1 to 1:3, and may be 1:2 in one embodiment.

The nitrogen source included in the fermentation medium of step (1) may further comprise an inorganic nitrogen source such as ammonium chloride ($NH_4Cl$) or ammonium nitrate ($NH_4NO_3$) in addition to soy peptone and yeast extract, and in an embodiment, may include ammonium chloride at a concentration of 1 to 3 g/L.

The fermentation medium of step (1) may not include corn steep liquor (CSL) as a nitrogen source.

The fermentation medium of step (1) may include potassium dihydrogen phosphate ($KH_2PO_4$) or disodium hydrogen phosphate ($Na_2HPO_4$) as a phosphorus source, and in an embodiment, may include disodium hydrogen phosphate at a concentration of 4 to 12 g/L, 6 to 10 g/L, or 7 to 9 g/L.

The fermentation medium of step (1) may comprise at least inorganic salts including a metal cation selected from the group consisting of sodium, potassium, magnesium, iron, manganese, cobalt, copper, and zinc, and in an embodiment, may comprise calcium chloride ($CaCl_2$) and magnesium sulfate ($MgSO_4 \cdot 7H_2O$), specifically, calcium chloride at a concentration of 0.1 to 2.0 g/L, and magnesium sulfate at a concentration of 1 to 3 g/L.

The fermentation medium of step (1) may be a medium comprising a carbohydrate, yeast extract, soy peptone, ammonium chloride, disodium hydrogen phosphate, calcium chloride, and magnesium sulfate, and specifically, may be a medium comprising 10 to 50 g/L of carbohydrate, 6 to 40 g/L of soy peptone, 5 to 15 g/L of yeast extract, 1 to 3 g/L of ammonium chloride, 4 to 12 g/L of disodium hydrogen phosphate, 0.1 to 2.0 g/L of calcium chloride, and 1 to 3 g/L of magnesium sulfate, but is not limited thereto.

Among the culturing conditions of step (1), the culturing temperature may be 30 to 43°C, or 33 to 40°C, pH may be 6.0 to 8.0, or 6.5 to 7.5, and the stirring speed may be 500 to 1100 rpm, or 600 to 1000 rpm, but is not limited thereto.

The method for producing pectinase from a *Bacillus spp.* microorganism of the present disclosure comprises step (2): supplying the medium of step (1) with a feed medium comprising soy peptone and yeast extract as nitrogen sources.

The feed medium of step (2) may be supplied to the fermentation medium when the dissolved oxygen (DO) level of the fermentation broth in the fermentation medium of step (1) increases.

The feed medium of step (2) may be supplied to the fermentation medium when the glucose concentration in the fermentation medium of step (1) is 1 g/L or less.

The feed medium of step (2) may have a carbon source supply rate of 7 to 14 mL/hr, 8 to 14 mL/hr, 9 to 14 mL/hr, 7 to 13 mL/hr, 8 to 13 mL/hr, 9 to 13 mL/hr, 7 to 12 mL/hr, 8 to 12 mL/hr, or 9 to 12 mL/hr.

The feed medium of step (2) may be continuously supplied at the supply rate for 60 to 130 hours.

In the present disclosure, the term "feed medium" refers to a medium that supplies additional carbon sources, nitrogen sources, phosphates, vitamins, and the like to the fermentation medium in a fed-batch culture method, and may be used interchangeably with the term "nutrient (feed) medium" herein.

The feed medium of step (2) may be a medium comprising soy peptone and yeast extract as nitrogen sources to increase the produced amount of pectinase from the *Bacillus spp.* microorganism or to increase the activity of pectinase produced from the *Bacillus spp.* microorganism.

The content of soy peptone included in the feed medium of step (2) may be 30 g/L or more, 40 g/L or more, 45 g/L or more, 50 g/L or more, 30 to 70 g/L, 35 to 70 g/L, 40 to 70 g/L, 45 to 70 g/L, 47 to 70 g/L, 30 to 65 g/L, 35 to 65 g/L, 40 to 65 g/L, 45 to 65 g/L, 47 to 65 g/L, 30 to 60 g/L, 35 to 60 g/L, 40 to 60 g/L, 45 to 60 g/L, 47 to 60 g/L, 30 to 55 g/L, 35 to 55 g/L, 40 to 55 g/L, 45 to 55 g/L, 47 to 55 g/L, 30 to 53 g/L, 35 to 53 g/L, 40 to 53 g/L, 45 to 53 g/L, 47 to 53 g/L, or 49 to 51 g/L.

The content of yeast extract included in the feed medium of step (2) may be 5 to 15 g/L, 7.5 to 15 g/L, 9 to 15 g/L, 9.5 to 15 g/L, 5 to 12.5 g/L, 7.5 to 12.5 g/L, 9 to 12.5 g/L, 9.5 to 12.5 g/L, 5 to 11 g/L, 7.5 to 11 g/L, 9 to 11 g/L, 9.5 to 11 g/L, 5 to 10.5 g/L, 7.5 to 10.5 g/L, 9 to 10.5 g/L, or 9.5 to 10.5 g/L.

In an embodiment of the present disclosure, when being cultured by supplying in a feed medium containing soy peptone at a concentration of 10 g/L or more based on a yeast extract concentration of 10 g/L contained in the feed medium, the relative activity of pectinase produced from the *Bacillus spp.* microorganism was 95% or more. When being cultured by supplying in a feed medium containing soy peptone at a concentration of 20 g/L or more, the relative activity was 100% or more, 105% or more, 110% or more, 115% or more, 120% or more, 125% or more, or 129% or more. When being cultured by supplying in a feed medium containing soy peptone at a concentration of 30 g/L or more, the relative activity was 130% or more, 133% or more, 136% or more, 137% or more, or 138% or more. When being cultured by supplying in a feed medium containing soy peptone at a concentration of 40 g/L or more, the relative activity was 140% or more, 143% or more, 146% or more, 149% or more, 152% or more, or 153% or more. When being cultured by supplying in a feed medium containing soy peptone at a concentration of 50 g/L or more, the relative activity was confirmed to increase by 155% or more, 160% or more, 165% or more, 170% or more, 175% or more, 180% or more, 185% or more, 190% or more, or 191% or more.

Therefore, the feed medium of the step (2) may contain soy peptone and yeast extract in a mixing ratio of 7:1 to 3:1, 6:1 to 4:1, 5.5:1 to 4.5:1, or specifically 5:1.

The feed medium of step (2) may not include ammonium chloride ($NH_4Cl$) as a nitrogen source

The feed medium of step (2) may comprise a carbohydrate as a carbon source, and the carbon source may be at least one selected from the group consisting of glucose, fructose, maltose, galactose, mannose, sucrose, arabinose, xylose, and glycerol, and in an embodiment, the carbon source may be maltose.

The content of carbon source included in the feed medium of step (2) may be 100 to 400 g/L, 150 to 400 g/L, 200 to 400 g/L, 220 to 400 g/L, 240 to 400 g/L, 100 to 350 g/L, 150 to 350 g/L, 200 to 350 g/L, 220 to 350 g/L, 240 to 350 g/L, 100 to 300 g/L, 150 to 300 g/L, 200 to 300 g/L, 220 to 300 g/L, 240 to 300 g/L, 100 to 280 g/L, 150 to 280 g/L, 200 to 280 g/L, 220 to 280 g/L, 240 to 280 g/L, 100 to 260 g/L, 150 to 260 g/L, 200 to 260 g/L, 220 to 260 g/L, 240 to 260 g/L, or 245 to 255 g/L.

The feed medium of step (2) may be a medium including carbohydrate, soy peptone, and yeast extract, and specifically, may be a medium including 100 to 400 g/L of carbohydrate, 40 to 60 g/L of soy peptone, and 5 to 15 g/L of yeast extract

The method for producing pectinase from a *Bacillus spp.* microorganism of the present disclosure may involve adjusting a concentration of soy peptone as a nitrogen source, included in the fermentation medium of step (1) and the feed medium of step (2) to increase the produced amount of pectinase from the *Bacillus spp.* microorganism or to increase the activity of pectinase produced from the *Bacillus spp.* microorganism. Specifically, the concentration ratio of soy peptone included in the fermentation medium to soy peptone included in the feed medium may be 2:3 to 7, 2:4 to 6, or 2:4.5 to 6.5, and in an embodiment, may be 2:5

The method for producing pectinase from a *Bacillus spp.* microorganism, comprising steps (1) and (2) of the present disclosure, can increase the activity of pectinase produced from the *Bacillus spp.* microorganism, promote the growth of the *Bacillus spp.* microorganism, and induce the production of pectinase from the *Bacillus spp.* microorganism.

The pectinase produced by the method for producing pectinase from a *Bacillus spp.* microorganism, comprising steps (1) and (2), may have an enzyme activity increased by 250% or more, 300% or more, 310% or more, 320% or more, 325% or more, or 330% or more, and in one embodiment, may be increased by 333% or more, compared to pectinase produced by culturing in a fermentation medium and feed medium that do not contain soy peptone as a nitrogen source.

The pectinase produced by the method for producing pectinase from a *Bacillus spp.* microorganism comprising steps (1) and (2), may have an enzyme activity increased by 2.5 times or more, 3 times or more, 3.5 times or more, 4 times or more, 4.1 times or more, 4.2 times or more, or 4.3 times or more, compared to the pectinase produced by culturing in a fermentation medium and feed medium that do not contain soy peptone as a nitrogen source.

Additionally, another aspect of the present disclosure provides a composition for producing pectinase, comprising a fermentation medium and/or a feed medium including both soy peptone and yeast extract as nitrogen sources.

The pectinase may be produced from a *Bacillus spp.* microorganism.

The *Bacillus spp*. microorganism, pectinase, fermentation medium, feed medium, soy peptone, yeast extract, and the like are as described above.

The composition may further comprise any suitable excipients commonly used in composition for enzyme production, in which the excipients may include, for example, preservatives, wetting agents, dispersing agents, suspending agents, buffering agents, stabilizers, or isotonic agents, but are not limited thereto.

[ADVANTAGEOUS EFFECTS]

The present invention relates to a medium composition for improving pectinase production of *Bacillus spp.* strains and a method for producing pectinase using the same. When *Bacillus subtilis* strains are cultured in a fermentation medium and feed medium including soy peptone and yeast extract as nitrogen sources in specific ratios according to the present invention, it was confirmed that pectate lyase activity was increased by 4 times or more compared to conventional *Bacillus spp.* strain culture media. Therefore, the medium composition and the method for producing pectinase using the same according to the present invention can be usefully applied for mass production of pectinase.

[BRIEF DESCRIPTION OF THE DRAWING]

FIG. 1 is a drawing representing the cell concentration in the culture broth over time after culturing *Bacillus spp.* strains in a basal medium at 37°C and pH 6.4 in fed-batch culture.

FIG. 2 is a drawing representing the specific components and their contents (%) of the basal medium (left table) and the medium optimized for organic nitrogen sources (right table) used in the experiment to test the effect of increased activity of pectate lyase produced from *Bacillus subtilis* strains.

FIG. 3 is a drawing representing the pectate lyase activity produced over time according to differences in the nitrogen source composition of the fermentation medium and feed medium in fed-batch culture of *Bacillus spp.* strains.

[MODE FOR INVENTION]

Hereinafter, the present invention will be described in more detail by way of the following examples. However, these are merely illustrative of the present invention, and the scope of the present invention is not limited by these examples.

**<Preparation of a strain producing pectate lyase>**

The strain producing pectate lyase was prepared by transforming a plasmid (SEQ ID NO: 2) expressing the pectate lyase gene (*pelA*) derived from *Bacillus licheniformis* into *Bacillus subtilis* LB700 strain (Accession No.: KCTC 18039P) (hereinafter referred to as BPL1). The amino acid sequence of *pelA* enzyme is shown in SEQ ID NO: 1.

The *Bacillus subtilis* BPL1 strain was streaked on TSB agar medium (enzymatic digest of casein 17.0 g, enzymatic digest of soybean meal 3.0 g, NaCl 5.0 g, dipotassium phosphate 2.5 g, dextrose 2.5 g, agar 15.0 g, 7.3±0.2 of final pH at 25°C) and cultured at 37°C for 12 hours to activate the strain.

**<Flask culture method>**

*Bacillus subtilis* BPL1 strain was inoculated with 1% of the seed suspension into a pre-prepared TSB medium (enzymatic digest of casein 17.0 g, enzymatic digest of soybean meal 3.0 g, NaCl 5.0 g, dipotassium phosphate 2.5 g, dextrose 2.5 g, agar 15.0 g, 7.3±0.2 of final pH at 25°C), and then cultured with shaking at 180 rpm at 37°C.

**<Feb-batch culture>**

Culturing of BPL1 strain using fed-batch culture was performed by consistently supplying the carbon source in the feed medium at a rate of 9 to 12 mL/hr, when the DO (dissolved oxygen) level began to increase with using DO as an indicator. The feed medium was supplied using a peristaltic pump. At this time, the components of fermentation medium were composed of 10.0 g/L maltose, 20.0 g/L glucose, 10.0 g/L yeast extract, 15 g/L corn steep liquor, 1.0 g/L calcium chloride, 8.0 g/L disodium hydrogen phosphate, 2.0 g/L ammonium chloride, and 2 g/L magnesium sulfate. The feed medium was composed of 250 g/L maltose, 10.0 g/L yeast extract, and 2.0 g/L ammonium chloride. Meanwhile, the fermentation conditions maintained a culture temperature of 37°C, and the pH was maintained at 6.7-7.2 using ammonia water. The stirring speed was increased from 600 rpm to 1,000 rpm to prevent dissolved oxygen (DO) limitation.

**<Method for measuring pectate lyase activity>**

One unit of pectate lyase activity is defined as an amount of enzyme required to release 1 μmol of unsaturated polygalacturonic acid (equivalent to D-galacturonic acid) per minute from polygalacturonic acid at 50°C and pH 7.73.

Pectate lyase acts on polygalacturonic acid (PGA) and forms an unsaturated bond at the non-reducing end of oligogalacturonic acids generated via β-elimination. The newly formed unsaturated (olefinic) bond exhibits strong absorption at 235 nm, which is utilized to measure the activity of pectate lyase.

One standard unit (U) of enzyme activity is defined as the amount of enzyme required to cleave polygalacturonic acid and produce 1 μmol of unsaturated polygalacturonic acid per minute. The unsaturated galacturonic acid is equivalent to D-galacturonic acid. The amount of enzyme needed to release 1 μmol of unsaturated polygalacturonic acid per minute from a 6 mg/mL polygalacturonic acid solution at 50°C and pH 7.73 is defined as 1(one) activity unit (U).

Specifically, pectate lyase activity was determined by measuring the absorbance at a wavelength of 235 nm of unsaturated polygalacturonic acid (unsaturated PGA) produced from the degradation of polygalacturonic acid (PGA) under conditions of 50°C and pH 7.73. The Tris buffer used for the activity assay was composed of 12.1 g/L Tris-base, 0.11 g/L CaCl2, and 1 g/L PEG6000, with the pH adjusted to 7.73 using HCl. The substrate was prepared by dissolving sodium polygalacturonate (Sigma #P3580) in the Tris buffer at a concentration of 6 mg/ml.

The enzyme reaction was carried out by adding 25 μL of enzyme to 500 μL of substrate, mixing well, and then incubating at 50°C for 15 minutes. Afterwards, 475 μL of 0.05 M phosphate solution was added to stop the reaction, and the absorbance at 235 nm was measured using a quartz cuvette. The confidence interval for absorbance was 0.2-0.4; if the absorbance value was outside this interval, the enzyme reaction was repeated with a different dilution factor of the enzyme.

To convert the absorbance to the concentration of unsaturated polygalacturonic acid, a standard curve was obtained, and the concentration of the product generated by the enzyme reaction could be calculated based on the standard curve. Based on this, the enzyme activity was calculated, and the formula used to determine pectate lyase activity is shown below.

[Calculation formula 1]

$$\text{Activity of pectate lyase (U/mL)} = C \text{ X } (1/T) \text{ X } (V_0/V_1) \text{ X } N$$

*C: Concentration of product

*$V_0$: Total reaction volume (1000 uL)
*$V_1$: Volume of enzyme added (25 uL)
*T: Reaction time (15 min)
*N: Dilution factor

**EXAMPLE 1. Activity analysis of the enzyme produced from *Bacillus subtilis* BPL1 in a basal fermentation medium**

After culturing *Bacillus subtilis* BPL1 strain in a basic fermentation medium without optimizing nitrogen source, the enzyme activity was evaluated.

Specifically, *Bacillus subtilis* BPL1 strain was streaked on TSB agar medium (enzymatic digest of casein 17.0 g, enzymatic digest of soybean meal 3.0 g, NaCl 5.0 g, dipotassium phosphate 2.5 g, dextrose 2.5 g, agar 15.0 g, 7.3±0.2 of final pH at 25°C) and cultured at 37°C for 12 hours to activate the strain. The activated *Bacillus subtilis* BPL1 strain was inoculated at 1% of seed culture suspension into pre-prepared TSB medium (enzymatic digest of casein 17.0 g, enzymatic digest of soybean meal 3.0 g, NaCl 5.0 g, dipotassium phosphate 2.5 g, dextrose 2.5 g, agar 15.0 g, 7.3±0.2 of final pH at 25°C) and shake-cultured at 37°C and 180 rpm. Then, the shake-cultured broth was inoculated into a fermentation medium containing 10.0 g/L maltose, 20.0 g/L glucose, 10.0 g/L yeast extract, 15 g/L corn steep liquor, 1.0 g/L calcium chloride, 8.0 g/L disodium hydrogen phosphate, 2.0 g/L ammonium chloride, and 2 g/L magnesium sulfate, to a final concentration of 5-20% of the fermentation medium volume. Culturing was performed for 76 or 77 hours, maintaining the culture temperature at 37°C and pH at 6.7-7.2 using ammonia water. After culturing, the supernatant of the culture broth was used as a crude enzyme solution, and the activity of pectate lyase was measured by the method described in < Method for measuring pectate lyase activity>, and the results are shown in FIG. 1 and Table 1.

[Table 1]

| No. | Culture time(hr) | O.D (600nm) | pH | Temp (°C) | Enzyme Activity (IU/ml) | |
|---|---|---|---|---|---|---|
| | | | | | batch | average |
| 1 | 76 | 69.2 | 6.7-7.2 | 37 | 29770 | 30214 |
| 2 | 77 | 69.2 | | | 30436 | |
| 3 | 77 | 70.5 | | | 30436 | |

As a result, as shown in FIG. 1 and Table 1, the pectate lyase produced by culturing *Bacillus subtilis* BPL1 strain in a basal fermentation medium without optimizing the nitrogen source, at a culture temperature of 37°C and pH 6.7-7.2, was confirmed to have an average activity of 30214 U/mL.

**EXAMPLE 2. Selection of organic nitrogen sources for high-concentration production of pectate lyase from *Bacillus subtilis* strains**

In fed-batch culture of *Bacillus subtilis* BPL1 strain, the following experiment was performed to test the effect of organic nitrogen sources on the activity of pectate lyase produced from BPL1 strain.

Specifically, yeast extract, peptone, beef extract, casein peptone, soy peptone, soybean meal, tryptone, corn steep liquor, or soy peptone as an organic nitrogen source was added to be 1% of concentration to TSB medium (enzymatic digest of casein 17.0 g, enzymatic digest of soybean meal 3.0 g, NaCl 5.0 g, dipotassium phosphate 2.5 g, dextrose 2.5 g, agar 15.0 g) as the fermentation medium in a flask, and cultured at 37°C and 200 rpm for 48 hours. The recovered culture broth was centrifuged, and the supernatant was used to measure pectate lyase activity by the method described in < Method for measuring pectate lyase activity>, and the results are shown in Table 2 below.

[Table 2]

| Organic Nitrogen Source (1.0%) | Enzyme Activity (U/ml) |
|---|---|
| Yeast extract | 16950 |
| Peptone | 17310 |
| Beef extract | 10740 |
| Casein peptone | 17010 |
| **Soy peptone** | **20030** |

(continued)

| Organic Nitrogen Source (1.0%) | Enzyme Activity (U/ml) |
|---|---|
| Soybean meal | 16745 |
| Tryptone | 12340 |
| Corn steep liquor | 9722 |
| Bacto peptone | 15012 |
| None | 11510 |

As a result, as shown in Table 2, the pectate lyase produced from *Bacillus subtilis* BPL1 strain cultured in a fermentation medium supplemented with 1% of soy peptone, showed 74% increase in enzyme activity compared to the control group without additional organic nitrogen sources, confirming that it exhibited the highest activity among the organic nitrogen sources. In contrast, the medium supplemented with corn steep liquor (CSL) showed lower activity than the control group.

**EXAMPLE 3. Confirmation of optimal concentration of soy peptone in fermentation medium for high-concentration production of pectate lyase from *Bacillus subtilis* strains in fed-batch culture**

Through Example 2, it was confirmed that a fermentation medium supplemented with soy peptone as an organic nitrogen source was the optimal condition for increasing the activity of pectate lyase produced from *Bacillus subtilis* strains in fed-batch culture. Accordingly, the following experiment was performed to determine the optimal concentration of soy peptone to be added to the fermentation medium for high-concentration production of pectate lyase from *Bacillus subtilis* strains. Meanwhile, in Example 2, corn steep liquor (CSL) resulted in low enzyme activity and thus was excluded from the fermentation medium.

Specifically, in a fermentation medium composition containing 10.0 g/L maltose, 20.0 g/L glucose, 10.0 g/L yeast extract, 15 g/L corn steep liquor, 1.0 g/L calcium chloride, 8.0 g/L disodium hydrogen phosphate, 2.0 g/L ammonium chloride, and 2 g/L magnesium sulfate, corn steep liquor was excluded, and soy peptone was added at concentrations of 5, 10, 20, 30, and 50 g/L, respectively, followed by culturing at 37°C for 90 hours. From 4 hours after culturing, DO increased, and feed medium was continuously supplied at a rate of 12 mL/hr. Except for the fermentation medium composition and condition of feed medium supply, other culture conditions and fermentation methods were the same as those described in <Feb-batch culture>. After culturing, the recovered culture broth was centrifuged, and the supernatant was used to measure pectate lyase activity by the method described in < Method for measuring pectate lyase activity>, and the results are shown in Table 3 below.

[Table 3]

| **Experimental Condition** | Culture Time (hr) | Cell Concentration (O.D600) | Enzyme Activity (IU/mL) | Relative Activity (%) |
|---|---|---|---|---|
| Control (Medium of Example 1) | 82.7 | 66.5 | 29603 | 0 |
| Fermentation medium (5 g/L soy peptone) | 82.9 | 67.2 | 30936 | 5 |
| Fermentation medium (10 g/L soy peptone) | 89.3 | 66.5 | 48440 | 64 |
| Fermentation medium (20 g/L soy peptone) | 90.7 | 67.9 | 53575 | 81 |
| Fermentation medium (30 g/L soy peptone) | 91.8 | 73.4 | 39739 | 34 |
| Fermentation medium (50 g/L soy peptone) | 91.8 | 72.6 | 32082 | 8 |

As a result, as shown in Table 3, pectate lyase produced from *Bacillus subtilis* strains showed maximum activity under the condition of addition of 20 g/L of soy peptone. The activity of pectate lyase was improved by 81% compared to the control when 20 g/L soy peptone was added, and it was confirmed that enzyme activity decreased from the condition of 30 g/L of added soy peptone.

By reflecting the results, a medium containing 10.0 g/L maltose, 20.0 g/L glucose, 10.0 g/L yeast extract, 20 g/L soy peptone, 1.0 g/L calcium chloride, 8.0 g/L disodium hydrogen phosphate, 2.0 g/L ammonium chloride, and 2 g/L magnesium sulfate was initially selected as the fermentation medium for *Bacillus subtilis* strains. A medium containing 250 g/L maltose, 10.0 g/L yeast extract, and 2.0 g/L ammonium chloride was initially selected as the feed medium. These were referred to as the adjusted medium of Example 3(hereinafter, the adjusted medium of Example 3).

**Example 4. Confirmation of optimal concentration of soy peptone in feed medium for high-concentration production of pectate lyase from *Bacillus subtilis* strains in fed-batch culture**

Fed-batch culture is a method of microbial culture in which new culture medium, growth-limiting substrate solution, or nutrients that limit the growth of microorganisms, such as amino acids, vitamins, phosphate, nitrogen sources, trace metal ions, and carbon sources, are supplied and controlled during the batch culture operation, so that the concentration of nutrients can be maintained at an appropriate level during the culture period. To determine the optimal concentration of soy peptone, an organic nitrogen source derived from Example 2 and Example 3, to be added to the feed medium in the fed-batch culture method, the following experiment was performed. This was to evaluate changes in pectate lyase production in the culturing of *Bacillus subtilis* BPL1 strain with additional increments of soy peptone.

Specifically, fed-batch culturing was performed by the method described in Example 3, except for the condition of adding soy peptone to the feed medium in the adjusted medium of Example 3 at concentrations of 10, 20, 30, 40, and 50 g/L, respectively. After culturing, the recovered culture broth was centrifuged, and the supernatant was used to measure pectate lyase activity by the method described in < Method for measuring pectate lyase activity>, and the results are shown in Table 4 below.

[Table 4]

| Experimental Condition | Culture Time (hr) | Cell Concentration ($O.D_{600}$) | Enzyme Activity (IU/mL) | Relative Activity (%) |
|---|---|---|---|---|
| Feed (no addition of soy peptone) | 102 | 66.5 | 56739 | 0 |
| Feed (10 g/L Soy peptone) | 101.5 | 67.2 | 110739 | 95 |
| Feed (20 g/L Soy peptone) | 98.2 | 66.5 | 129830 | 129 |
| Feed (30 g/L Soy peptone) | 88.9 | 67.9 | 135253 | 138 |
| Feed (40 g/L Soy peptone) | 100.5 | 66.5 | 143387 | 153 |
| Feed (50 g/L Soy peptone) | 114 | 67.9 | 165253 | 191 |

As a result, as shown in Table 4, it was confirmed that the activity of pectate lyase produced from *Bacillus subtilis* strains increased proportionally with higher concentrations of soy peptone in the feed medium, showing maximum activity at a soy peptone concentration of 50 g/L. Furthermore, the method of supplying the fermentation medium and the supply medium in a certain ratio showed a pattern that greatly contributed to the effect of increasing the activity of pectate lyase. However, 2.0 g/L ammonium chloride was excluded from the feed medium during culturing due to the increase in organic nitrogen sources.

By reflecting the results, a medium containing 10.0 g/L maltose, 20.0 g/L glucose, 10.0 g/L yeast extract, 20 g/L soy peptone, 1.0 g/L calcium chloride, 8.0 g/L disodium hydrogen phosphate, 2.0 g/L ammonium chloride, and 2 g/L magnesium sulfate was secondarily selected as the fermentation medium for *Bacillus subtilis* strains. A medium containing 250 g/L maltose, 10.0 g/L yeast extract, and 50 g/L soy peptone was secondarily selected as the feed medium. There were referred to as the adjusted medium of Example 4 (hereinafter, the adjusted medium of Example 4).

**Example 5. Confirmation of increased activity of pectate lyase produced from *Bacillus subtilis* strains in medium optimized with organic nitrogen source**

The activity of pectate lyase produced from *Bacillus subtilis* strains was compared between a medium optimized with an organic nitrogen source for high-concentration production of pectate lyase derived from the fed-batch culture in Example 4 and a basal medium without optimization of organic nitrogen source.

Specifically, *Bacillus subtilis* BPL1 strain was cultured twice using fed-batch culture in the basal medium of Example 1 and the medium optimized with organic nitrogen sources of Example 4, respectively. The differences in composition and their contents between the basal medium of Example 1 and the medium optimized with organic nitrogen sources of Example 4 are shown in FIG. 2. Culture was performed by preparing media such that the total content of each component became 100% by adding distilled water to the components listed in FIG. 2. After culturing, the recovered culture broth was centrifuged, and the supernatant was used to measure pectate lyase activity by the method described in < Method for measuring pectate lyase activity>, and the results are shown in Table 5 and FIG. 3 below.

[Table 5]

| Experimental Condition | Culture Time (hr) | Cell Concentration ($O.D_{600}$) | Enzyme Activity (IU/mL) | Relative Activity (%) |
|---|---|---|---|---|
| Medium composition (1) of EXAMPLE 1 | 114 | 66.5 | 36739 | 33739 |
| Medium composition (2) of EXAMPLE 1 | 114 | 67.2 | 30739 | |
| Medium composition (1) of EXAMPLE 4 | 114 | 66.5 | 137387 | 146320 |
| Medium composition (2) of EXAMPLE 4 | 114 | 67.9 | 168040 | |

As a result, it was confirmed that pectate lyase produced from *Bacillus subtilis* strains showed the maximum activity under conditions where the addition ratio of soy peptone to yeast extract in the fermentation medium was 2:1, and the addition ratio of soy peptone to yeast extract in the feed medium was 5: 1. The activity of the produced pectate lyase was significantly improved by 333%, or approximately 4.3 times or more, compared to the basal medium without soy peptone.

From the foregoing description, those skilled in the art to which the present disclosure pertains will understand that the present disclosure can be implemented in other specific forms without changing its technical spirit or essential features. In this regard, the examples described above are to be understood as illustrative in all respects and not restrictive. The scope of the present disclosure should be interpreted as including all changes or modified forms derived from the meaning and scope of the claims described below and their equivalent concepts, rather than from the detailed description above.

## Claims

**1.** A method for producing pectinase from *Bacillus spp.* microorganism, the method comprising:

(1) culturing *Bacillus spp.* microorganisms in a fermentation medium comprising soy peptone and yeast extract as nitrogen sources; and
(2) supplying the medium of step (1) with a feed medium comprising soy peptone and yeast extract as nitrogen sources.

**2.** The method for producing pectinase according to claim 1, wherein the *Bacillus spp*. microorganism is *Bacillus subtilis.*

**3.** The method for producing pectinase according to claim 1, wherein the pectinase is a pectate lyase.

**4.** The method for producing pectinase according to claim 1, wherein a content of the soy peptone in the fermentation medium of step (1) is 10 to 30 g/L.

**5.** The method for producing pectinase according to claim 1, wherein a mixing ratio of the soy peptone to the yeast extract in the fermentation medium of step (1) is 3:1 to 1: 1.

**6.** The method for producing pectinase according to claim 1, wherein the fermentation medium of step (1) does not contain corn steep liquor as a nitrogen source.

**7.** The method for producing pectinase according to claim 1, wherein a content of the soy peptone in the feed medium of step (2) is 40 to 60 g/L.

**8.** The method for producing pectinase according to claim 1, wherein a mixing ratio of the soy peptone to the yeast extract in the feed medium of step (2) is from 6:1 to 4:1.

**9.** The method for producing pectinase according to claim 1, wherein the feed medium of step (2) is supplied to the fermentation medium when the dissolved oxygen (DO) level in the fermentation broth increases.

**10.** The method according to claim 1, wherein a feed rate of carbon source in the feed medium of step (2) is 7 to 14 mL per hour.

11. A composition for producing pectinase, comprising a fermentation medium or a feed medium, wherein the fermentation medium or the feed medium comprises both soy peptone and yeast extract as nitrogen sources.

12. The composition for producing pectinase according to claim 10, wherein the pectinase is produced from a *Bacillus spp.* microorganism.

13. The composition for producing pectinase according to claim 10, wherein the pectinase is a pectate lyase.

14. The composition for producing pectinase according to claim 10, wherein a content of the soy peptone in the fermentation medium is 10 to 30 g/L.

15. The composition for producing pectinase according to claim 10, wherein a content ratio of the soy peptone to the yeast extract in the fermentation medium is 1.5 to 2.5: 1.

16. The composition for producing pectinase according to claim 10, wherein the fermentation medium does not contain corn steep liquor as a nitrogen source.

17. The composition for producing pectinase according to claim 10, wherein a content of the soy peptone in the feed medium is 40 to 60 g/L.

18. The composition for producing pectinase according to claim 10, wherein a mixing ratio of the soy peptone to the yeast extract in the feed medium is from 6:1 to 4:1.

【FIG. 1】

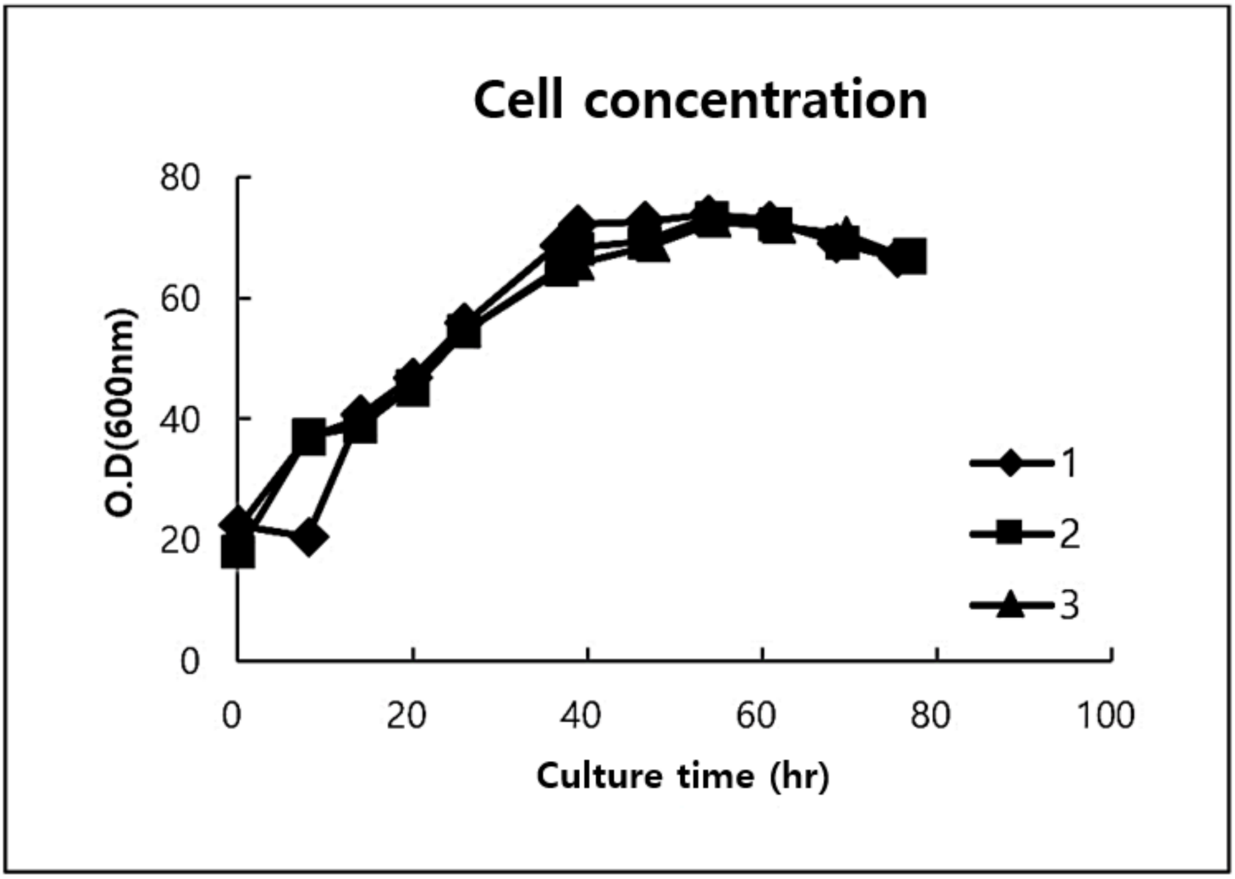
Cell concentration
O.D(600nm)
80
60
40
20
0
0
20
40
60
80
100
Culture time (hr)
1
2
3

【FIG. 2】

| Type | Basal medium | Content(%) |
|---|---|---|
| Main | Maltose | 10 |
| | Glucose | 20 |
| | yeast extract (powder) | 1 |
| | C.S.L | 1.5 |
| | CaCl2 | 0.1 |
| | NaH2PO4 | 0.8 |
| | NH4Cl | 0.2 |
| | MgSO40-7H2O | 0.2 |
| Feed | Maltose | 25 |
| | yeast extract (powder) | 1 |
| | NH4Cl | 0.2 |

| Type | Optimal medium | Content(%) |
|---|---|---|
| Main | Maltose | 10.0 |
| | Glucose | 20.0 |
| | yeast extract (powder) | 1.0 |
| | Soypeptone | 2.0 |
| | CaCl2 | 0.1 |
| | NaH2PO4 | 0.8 |
| | NH4Cl | 0.2 |
| | MgSO40-7H2O | 0.2 |
| Feed | Maltose | 25 |
| | yeast extract (powder) | 1 |
| | Soypeptone | 5 |

【FIG. 3】

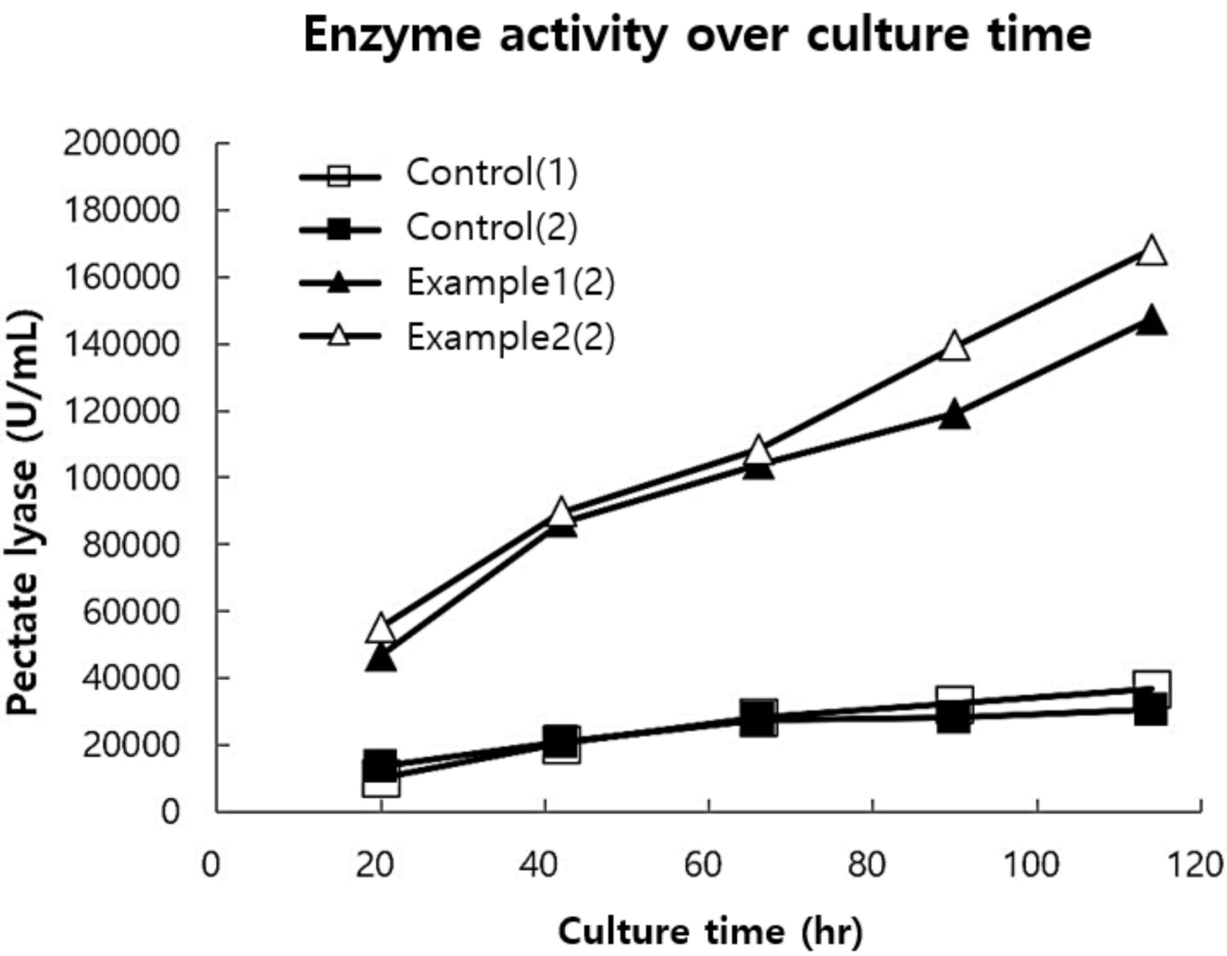
Enzyme activity over culture time
Pectate lyase (U/mL)
200000
180000
160000
140000
120000
100000
80000
60000
40000
20000
0
Control(1)
Control(2)
Example1(2)
Example2(2)
0
20
40
60
80
100
120
Culture time (hr)

## INTERNATIONAL SEARCH REPORT

International application No.
**PCT/KR2024/006294**

**A. CLASSIFICATION OF SUBJECT MATTER**

**C12N 9/24**(2006.01)i; **C12N 1/20**(2006.01)i; **C12N 1/38**(2006.01)i

According to International Patent Classification (IPC) or to both national classification and IPC

**B. FIELDS SEARCHED**

Minimum documentation searched (classification system followed by classification symbols)

C12N 9/24(2006.01); C12N 1/00(2006.01); C12N 1/20(2006.01); C12N 9/00(2006.01); C12N 9/88(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 바실러스(Bacillus), 펙티나아자(pectinase), 발효(fermentation), 소이펩톤(soy peptone), 효모 추출물(yeast extract)

**C. DOCUMENTS CONSIDERED TO BE RELEVANT**

| Category* | Citation of document, with indication, where appropriate, of the relevant passages | Relevant to claim No. |
|---|---|---|
| X | KASHYAP, D. R. et al. Enhanced production of pectinase by Bacillus sp. DT7 using solid state fermentation. Bioresource Technology. 2003, vol. 88, pp. 251-254.<br>See abstract; tables 3-4; and pages 252-253. | 1-18 |
| A | KR 10-2001-0077298 A (HWANG, Young Il) 17 August 2001 (2001-08-17)<br>See abstract; claims 1-4; and table 3. | 1-18 |
| A | CN 113046341 A (QINGDAO INSTITUTE OF BIOENERGY AND PROCESS, CHINESE ACADEMY OF SCIENCES) 29 June 2021 (2021-06-29)<br>See abstract; and claims 1-7. | 1-18 |
| A | KR 10-2005-0010434 A (THE INDUSTRY & ACADEMIC COOPERATION IN CHUNGNAM NATIONAL UNIVERSITY (IAC)) 27 January 2005 (2005-01-27)<br>See entire document. | 1-18 |
| A | KR 10-2002-0025396 A (IN BIONET. INC.) 04 April 2002 (2002-04-04)<br>See entire document. | 1-18 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **12 August 2024** | **13 August 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.
**PCT/KR2024/006294**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/KR2024/006294**

| Patent document cited in search report | | | Publication date (day/month/year) | Patent family member(s) | | | Publication date (day/month/year) |
|---|---|---|---|---|---|---|---|
| KR | 10-2001-0077298 | A | 17 August 2001 | KR | 10-0355619 | B1 | 11 October 2002 |
| CN | 113046341 | A | 29 June 2021 | None | | | |
| KR | 10-2005-0010434 | A | 27 January 2005 | KR | 10-0489451 | B1 | 17 May 2005 |
| KR | 10-2002-0025396 | A | 04 April 2002 | KR | 10-0437968 | B1 | 02 July 2004 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020060074794